# EUROPEAN PATENT APPLICATION

(11) **EP 4 740 829 A1**
(43) Date of publication of application: **13.05.2026**
(21) Application number: 23945663.5
(22) Date of filing: 15.11.2023
(51) Int. Cl.: A61B 1/018

(54) **BENDABLE THORACOSCOPE KIT WITH STRAIGHT WORKING CHANNEL**

(30) Priority: 20.07.2023 CN 202310890067
(71) Applicant: ZHEJIANG UE MEDICAL CORP., Taizhou, Zhejiang 317300 (CN)
(72) Inventor: HOU, Gang, Beijing 100029 (CN); DENG, Mingming, Beijing 100029 (CN)
(74) Representative: Chung, Hoi Kan
(86) International application number: PCT/CN2023/131828
(87) International publication number: WO 2025/015783

(57) **Abstract**

Disclosed is a flexible thoracoscope kit with a straight working channel. The thoracoscope kit includes rigid biopsy forceps (100) and a flexible thoracoscope (200) with a straight working channel. The rigid biopsy forceps (100) are provided with a forceps head (101), an elastic guide rod segment (102), and a rigid guide rod segment (103), and the forceps head (101) is connected to a first end of the rigid guide rod segment (103) through the elastic guide rod segment (102). An endoscope tube of the thoracoscope (200) includes a rigid tube segment (201) and a soft tube segment (202), and a rear end of the soft tube segment (202) is connected to a front end of the rigid tube segment (201). The forceps head (101), the elastic guide rod segment (102), and the rigid guide rod segment (103) are movably mounted in the endoscope tube, the elastic guide rod segment (102) at least partially overlaps with the soft tube segment (202), and the soft tube segment (202) can synchronously bend with the elastic guide rod segment (102) under the action of a control mechanism, to implement the steering of the forceps head (101). The thoracoscope kit improves the diagnosis and treatment efficacy of the flexible thoracoscope (200).

## Description

### TECHNICAL FIELD

This disclosure belongs to the technical field of thoracoscopes, and specifically relates to a flexible thoracoscope kit with a straight working channel.

### BACKGROUND

The pleural cavity is a closed potential cavity gap formed by the visceral and parietal layers of the pleura folding and transitioning at the root of the lung and is composed of the visceral pleura closely attached to the lung surface and the parietal pleura closely attached to the inner wall of the thoracic cage. Under normal conditions, there is a thin layer of liquid on the surfaces of both the visceral pleura and the parietal pleura, serving as a lubricant during respiratory movements. Any factor that causes rapid formation or slow absorption of liquid in the pleural cavity results in pleural effusion, which can affect the pulmonary ventilation function and the hemodynamic stability. Lung, pleural, and extrapulmonary diseases, and medications can all cause pleural effusion. Pleural effusion is a common complication of various diseases, and its etiological diagnosis is challenging.

Medical thoracoscopy (also known as pleuroscopy) is an invasive operation technique, which is a method that uses an electronic bronchoscope, a rigid or soft-rigid combined (semi-rigid) thoracoscope, and the like to insert into the pleural cavity through the chest wall and intercostal space to perform biopsy or treatment on lesions in the thoracic cavity under direct visualization. Studies have shown that the sensitivity of medical thoracoscopy biopsy in diagnosing MPE is 92.6% to 97%, and the specificity is 99% to 100%. Currently, the pathological examination of pleural biopsy tissue under medical thoracoscopy is the gold standard for diagnosing unexplained pleural effusion. Medical thoracoscopy can also provide endoscopic evidence for some cases of pleural effusion with unclear diagnoses, such as pleural effusion caused by rheumatoid arthritis, cirrhosis of the liver or pancreatitis, and some rare causes such as amyloidosis or sarcoidosis.

Medical thoracoscopy includes two types: rigid thoracoscopes and flexible thoracoscopes.

The existing rigid medical thoracoscope requires two incisions in the chest wall, which causes significant harm to the patients. Furthermore, the whole rigid medical thoracoscope is of a rigid tubular structure. After reaching into the diseased position of the human body, the operation of adjusting the angle is complicated, which brings inconvenience to the operator. Moreover, the angle adjustment range is limited, and sometimes it is impossible to reach the lesion to acquire tissue standard block samples, which has certain limitations.

The existing flexible thoracoscope can only use flexible biopsy tools, such as flexible biopsy forceps, for pleural biopsy operations, and the biopsy efficacy is lower than that of the rigid thoracoscope using rigid biopsy tools. This has been confirmed by research results published after peer review. The overall structure of flexible biopsy forceps is soft, and the flexible biopsy forceps extend from the side wall of the flexible thoracoscope, resulting in low biopsy efficacy of the flexible thoracoscope, small acquired tissue standard block samples, severe compressional deformation of tissue structures, long time consumption, difficulty in acquiring some tissues, and limited success rate and quality of sampling.

In summary, the biopsy efficacy of current medical thoracoscopy still cannot meet actual clinical requirements, which is a significant technical challenge for clinicians.

### SUMMARY

In view of the above analysis, embodiments of this disclosure aim to provide a flexible thoracoscope kit with a straight working channel, to solve the problem that the biopsy efficacy of existing medical thoracoscopy still cannot meet actual clinical requirements.

The purpose of this disclosure is achieved as follows:
A flexible thoracoscope kit with a straight working channel, including:
rigid biopsy forceps, where the rigid biopsy forceps are provided with a forceps head, an elastic guide rod segment, and a rigid guide rod segment, the forceps head is connected to a first end of the rigid guide rod segment through the elastic guide rod segment, a second end of the rigid guide rod segment is connected to a first control mechanism and a second control mechanism, the first control mechanism is configured to control opening and closing of the forceps head, and the second control mechanism is configured to control bending of the elastic guide rod segment, to implement steering of the forceps head; and
a thoracoscope, where the thoracoscope is provided with an endoscope tube, and the endoscope tube is provided with a hollow channel extending along an axial direction; and the endoscope tube includes a rigid tube segment and a soft tube segment, and a rear end of the soft tube segment is connected to a front end of the rigid tube segment; and
the forceps head, the elastic guide rod segment, and the rigid guide rod segment are movably mounted in the hollow channel, the elastic guide rod segment at least partially overlaps with the soft tube segment, and the soft tube segment can synchronously bend with the elastic guide rod segment.

Further, the second control mechanism includes a bending manipulation wire, one end of the bending manipulation wire is connected to a forceps base of the forceps head, the other end is located at a control end of the rigid biopsy forceps, and the bending of the elastic guide rod segment is implemented by driving the bending manipulation wire, thereby implementing the steering of the forceps head.

Further, the elastic guide rod segment has a first length, the soft tube segment has a second length, and the first length is greater than the second length.

Further, the first length is 1.5 to 2 times the second length.

Further, the elastic guide rod segment and the rigid guide rod segment are of an integral structure, and the elastic guide rod segment is a helical spring tube cut by a laser.

Further, the helical spring tube has a pitch, and the pitch progressively decreases from a middle portion of the helical spring tube towards two ends.

Further, the forceps head includes a forceps base, a first forceps jaw, and a second forceps jaw, and the forceps base is fixedly connected to the elastic guide rod segment; and the first forceps jaw and the second forceps jaw are disposed on the forceps base in an openable manner, and the first forceps jaw and the second forceps jaw form an accommodation space for accommodating tissue after being opened.

Further, the first forceps jaw is provided with first row teeth, and an end portion of the first row teeth is provided with a first end tooth; and the second forceps jaw is provided with second row teeth, and an end portion of the second row teeth is provided with a second end tooth; longitudinal cross sections of the first end tooth and the second end tooth are both triangles, a vertex angle of the first end tooth has a first angle bisector, and a vertex angle of the second end tooth has a second angle bisector; an included angle between the first forceps jaw and the second forceps jaw has a third angle bisector; and in a fully open state of the first forceps jaw and the second forceps jaw, included angles between the first angle bisector, the second angle bisector, and the third angle bisector are all α, where 0° ≤ α ≤ 5°.

Further, a middle portion of the first forceps jaw is provided with a first accommodation space, and the first row teeth are arranged around the first accommodation space; a middle portion of the second forceps jaw is provided with a second accommodation space, and the second row teeth are arranged around the second accommodation space; and after the first forceps jaw and the second forceps jaw are engaged, the first accommodation space and the second accommodation space jointly constitute the accommodation space for accommodating the tissue.

Further, at least one of the first forceps jaw and the second forceps jaw is provided with a liquid discharge hole, and the liquid discharge hole is communicated with the accommodation space.

Further, a front end of the soft tube segment is provided with an insertion head, and a front end of the insertion head is provided with a camera and a light source lamp.

Further, the thoracoscope is further provided with an operating main body, the operating main body is provided with a housing, a bottom portion of the housing is provided with a mounting opening, and the endoscope tube is connected to the mounting opening in a sealing manner; and a center of a top portion of the housing is provided with a biopsy channel opening, and a central line of the biopsy channel opening overlaps with an axis of the rigid tube segment.

Further, the operating main body is provided with a third control mechanism, and the third control mechanism is configured to control a normal operation of the thoracoscope.

Further, a mounting frame is disposed in a lower space of the housing, and the mounting frame is internally provided with a guide tube in a penetrating manner; a bottom portion of the insertion head is provided with a through hole 1 along a length direction thereof, and the through hole is communicated with the hollow channel of the endoscope tube; and one end of the endoscope tube is communicated with the through hole, and the other end is communicated with the guide tube.

Further, the biopsy channel opening at the top portion of the housing is provided with a mounting head of a hollow structure, and an opening at a top end of the mounting head is detachably provided with a sealing plug.

Further, a fastening frame is further disposed in an internal space of the housing, an adjustment disc is fixedly connected to the fastening frame, a guide wire is connected to the adjustment disc in a wound manner, two ends of the guide wire are both connected to the insertion head, a top end of the fastening frame extends into an upper space of the housing, a handle is disposed outside the housing, the handle is connected to the adjustment disc, and the guide wire is retracted and extended by rotating the adjustment disc with the handle, thereby controlling the bending of the soft tube segment.

Further, a soft tube is further connected to a side wall of the mounting head, and the soft tube is connected to an external pump body and is used for extracting a foreign object.

Further, the operating main body is further provided with a data interface, the data interface is connected to a computer through a data cable, and a data image of an operation process is transmitted to the computer through the data cable.

Compared with the prior art, this disclosure can at least implement one of the following beneficial effects:
a) According to the flexible thoracoscope kit with a straight working channel provided in this disclosure, an insertion portion of the forceps head of the rigid biopsy forceps includes the elastic guide rod segment and the rigid guide rod segment. The endoscope tube of the thoracoscope includes the rigid tube segment and the soft tube segment. The soft tube segment bends synchronously by controlling the bending of the elastic guide rod segment. This can conveniently and rapidly adjust the angle of the forceps head, to acquire a large tissue standard block sample, thereby improving a success rate of pleural tissue acquisition.
b) According to the flexible thoracoscope kit with a straight working channel provided in this disclosure, a biopsy channel of the thoracoscope is designed in a penetrating manner, and the biopsy channel opening thereof is in the center of the top portion of the operating main body. On the one hand, the rigid biopsy forceps and therapeutic tools can pass through the front flexible thoracoscope, improving the capability of grasping diseased tissue during biopsy and treatment. On the other hand, better coaxiality between the flexible rigid biopsy forceps and the thoracoscope body is ensured, reducing the impact of the operator rotating a wrist to adjust the thoracoscope body of the thoracoscope on the operation of the flexible rigid biopsy forceps. In addition, this has the advantage of a small single-port trauma, which has better effects than those of biopsy and treatment using traditional rigid tools.
c) According to the flexible thoracoscope kit with a straight working channel provided in this disclosure, when the first forceps jaw and the second forceps jaw of the forceps head are in the fully open state, the outermost end tooth of at least one forceps jaw can be inserted into the tissue at a vertical or near-vertical angle, which can improve the efficiency and effect of tissue acquisition.
d) According to the flexible thoracoscope kit with a straight working channel provided in this disclosure, the thoracoscope with a straight working channel can be compatible with a flexible biopsy/treatment tool and a rigid biopsy/treatment tool, both of which can pass through. The operating habits are similar to those of a traditional flexible thoracoscope, which are easy to learn and master. The addition of the rigid biopsy/treatment tool for passing through makes the biopsy and treatment efficacy thereof similar to that of a rigid thoracoscope, compensating for the insufficient efficacy of the flexible thoracoscope and the flexible biopsy/treatment tool.

### BRIEF DESCRIPTION OF THE DRAWINGS

To describe the technical solutions in the embodiments of the specification or in the prior art more clearly, the following briefly introduces the accompanying drawings required for describing the embodiments or the prior art. Apparently, the accompanying drawings in the following description show merely some embodiments recorded in the embodiments of the specification, and those of ordinary skill in the art may still derive other drawings from these accompanying drawings.
FIG. 1 is a schematic structural diagram of a flexible thoracoscope kit with a straight working channel according to this disclosure;
FIG. 2 is a schematic diagram of a flexible thoracoscope kit with a straight working channel whose front end is in a bending state according to this disclosure;
FIG. 3 is a schematic structural diagram of a thoracoscope according to this disclosure;
FIG. 4 is a schematic structural diagram of a soft tube segment that is not bent of a thoracoscope according to this disclosure;
FIG. 5 is a schematic structural diagram of a soft tube segment that is bent of a thoracoscope according to this disclosure;
FIG. 6 is a schematic exploded view of an operating main body of a thoracoscope according to this disclosure;
FIG. 7 is another schematic exploded view of an operating main body of a thoracoscope according to this disclosure;
FIG. 8 is a schematic structural diagram of rigid biopsy forceps according to this disclosure;
FIG. 9 is a schematic partial structural diagram of rigid biopsy forceps according to this disclosure; and
FIG. 10 is a schematic structural diagram of a forceps head of rigid biopsy forceps according to this disclosure.

Reference numerals:
100-rigid biopsy forceps; 101-forceps head; 1011-forceps base; 1012-first forceps jaw; 1012a-first end tooth; 1013-second forceps jaw; 1013a-second end tooth; 102-elastic guide rod segment; 103-rigid guide rod segment; 104-bending manipulation wire; 105-pulling ring; 106-first control mechanism;
200-thoracoscope; 201-rigid tube segment; 202-soft tube segment; 203-insertion head; 2031-through hole; 204-camera; 205-light source lamp; 206-operating main body; 207-housing; 2071-first housing; 2072-second housing; 208-mounting frame; 209-guide tube; 210-mounting head; 211-sealing plug; 212-fastening frame; 213-adjustment disc; 214-guide wire; 215-handle; 216-soft tube; 217-data interface; 218-first key; 219-second key.

### DETAILED DESCRIPTION OF THE EMBODIMENTS

To make the purposes, technical solutions, and advantages of the embodiments of this disclosure clearer, the following clearly and completely describes the technical solutions in the embodiments of this disclosure with reference to the accompanying drawings in the embodiments of this disclosure. Apparently, the described embodiments are some but not all of the embodiments of this disclosure. It should be noted that without conflict, the implementations of this disclosure and the features in the implementations can be combined, separated, interchanged, and/or rearranged with each other. All other embodiments obtained by those of ordinary skill in the art based on the embodiments of this disclosure without creative efforts shall fall within the protection scope of this disclosure.

In the accompanying drawings, for clarity and/or descriptive purposes, the size and relative sizes of the components may be exaggerated. When exemplary embodiments may be implemented in different ways, the specific process orders may be executed in an order different from that described. For example, two consecutively described processes may be simultaneously executed basically or executed in an order opposite to that described. In addition, the same reference numerals represent the same components.

The terms used herein are only for the purpose of describing the specific embodiments rather than for limiting the specific embodiments. As used herein, the singular forms "a/an" and "the" are intended to include the plural forms, unless the context clearly indicates otherwise. In addition, when the terms "comprise" and/or "include" and variations thereof are used in the specification, it indicates the existence of stated features, wholes, steps, operations, components, assemblies, and/or combinations thereof, but does not exclude the existence or addition of one or more other features, wholes, steps, operations, components, assemblies, and/or combinations thereof. It should also be noted that as used herein, the terms "basically", "approximately", and other similar terms are used as terms of approximation and not as terms of degree, and as such, are used to account for the inherent variations in measured, calculated, and/or provided values that those of ordinary skill in the art would recognize.

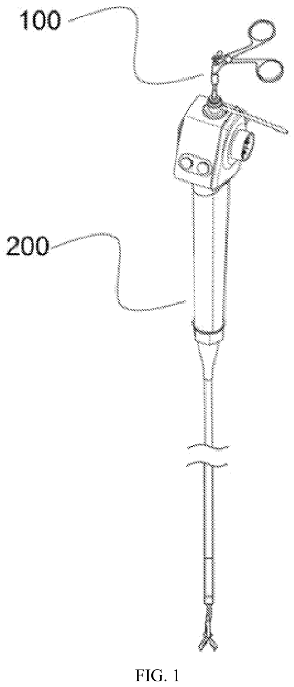

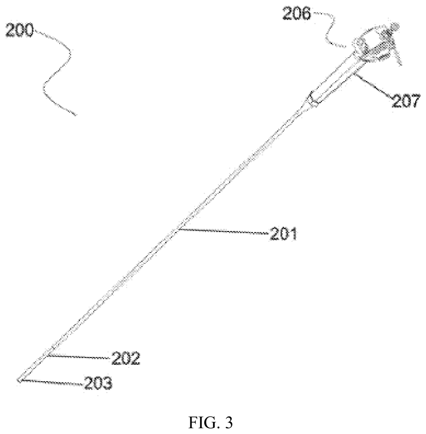

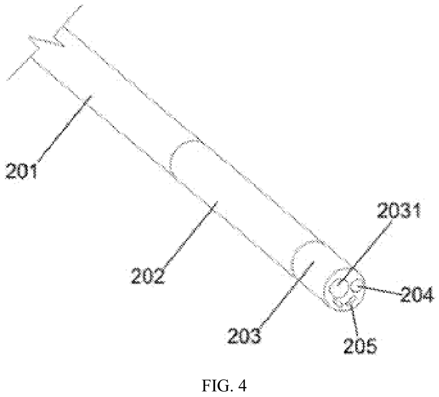

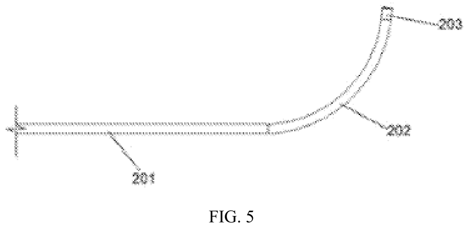

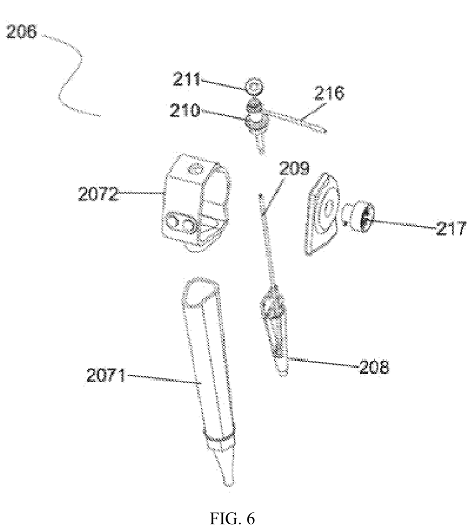

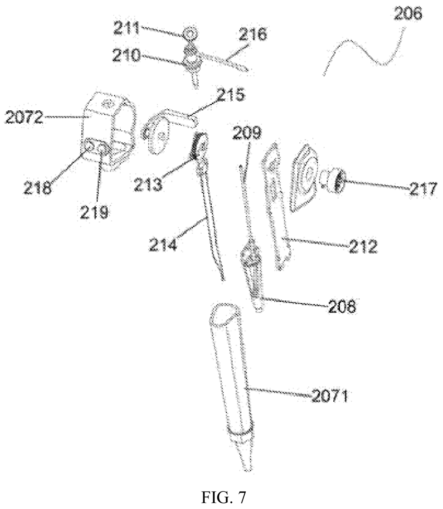

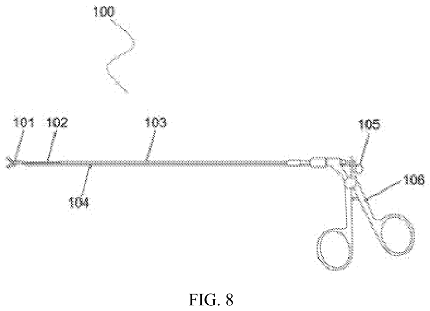

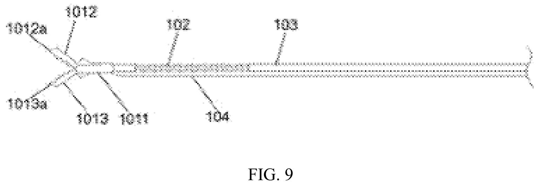

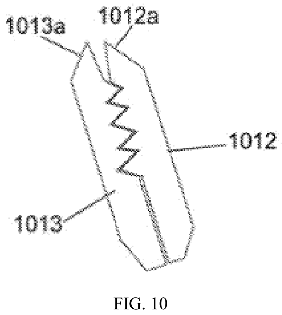

As shown in FIG. 1 to FIG. 10, a specific embodiment of this disclosure discloses a flexible thoracoscope kit with a straight working channel, which can be used for diagnosis and treatment of pleural diseases and includes rigid biopsy forceps 100 and a thoracoscope 200. The rigid biopsy forceps 100 are provided with a forceps head 101 and an insertion portion, and the forceps head 101 is connected to an end portion of the insertion portion. The insertion portion includes a rigid main body portion and a bendable portion located at a front end. The rigid main body portion cannot bend, and only a small segment at the front end can bend. Specifically, the insertion portion includes an elastic guide rod segment 102 and a rigid guide rod segment 103, and the elastic guide rod segment 102 and the rigid guide rod segment 103 jointly constitute the insertion portion. The forceps head 101 is connected to a first end of the rigid guide rod segment 103 through the elastic guide rod segment 102, and a second end of the rigid guide rod segment 103 is connected to a first control mechanism 106 and a second control mechanism. The first control mechanism 106 is configured to control the opening and closing of the forceps head 101; and the second control mechanism is configured to control the bending of the elastic guide rod segment 102, to implement the steering of the forceps head 101. The thoracoscope 200 is a flexible thoracoscope with a straight working channel, the thoracoscope 200 is provided with an endoscope tube, and the endoscope tube is provided with a hollow channel extending along an axial direction. The endoscope tube includes a rigid tube segment 201 and a soft tube segment 202, and a rear end of the soft tube segment 202 is connected to a front end of the rigid tube segment 201. The forceps head 101, the elastic guide rod segment 102, and the rigid guide rod segment 103 are movably mounted in the hollow channel, the elastic guide rod segment 102 at least partially overlaps with the soft tube segment 202, and the soft tube segment 202 can synchronously bend with the elastic guide rod segment 102.

During implementation, the forceps head 101 and the insertion portion of the rigid biopsy forceps 100 are mounted in the endoscope tube of the thoracoscope 200. An operator inserts the forceps head 101 into a designated position in the body of a patient and controls the bending of the elastic guide rod segment 102 according to a to-be-processed position by using the second control mechanism, to enable the forceps head 101 to bend towards a target position. In this process, the operator may integrally rotate the rigid biopsy forceps 100 and the thoracoscope 200, to enable the forceps head 201 to precisely rotate to the target position. After the forceps head is adjusted to the designated position, the forceps head 101 is closed by using the first control mechanism, to remove tissue at the target position.

In this embodiment, the thoracoscope 200 is further provided with an operating main body 206. The endoscope tube is connected to the operating main body, the operating main body 206 is provided with a housing 207, the center of a bottom portion of the housing 207 is provided with a mounting opening, and the endoscope tube is connected to the mounting opening in a sealing manner. The center of a top portion of the housing 207 is provided with a biopsy channel opening, and a central line of the biopsy channel opening overlaps with an axis of the rigid tube segment 201. In this way, the forceps head 101 and the insertion portion of the rigid biopsy forceps 100 can be coaxially inserted into the operating main body 206 and the endoscope tube of the thoracoscope 200, so that better coaxiality between the rigid biopsy forceps 100 and the operating main body 206 of the thoracoscope 200 can be ensured, reducing the impact of the operator rotating a wrist to adjust a thoracoscope body of the thoracoscope on the operation of the rigid biopsy forceps 100.

In this embodiment, the operating main body 206 is provided with a third control mechanism, and the third control mechanism is configured to control a normal operation of the thoracoscope 200. The first control mechanism 106 controlling the opening and closing of the forceps head 101 and the third control mechanism controlling the normal operation of the thoracoscope 200 can be implemented by using the prior art or with minor modifications based on the prior art, which are not described in detail again herein.

In this embodiment, the second control mechanism plays a role in controlling the bending of the elastic guide rod segment 102, as long as the structure can implement the bending of the elastic guide rod segment 102. The second control mechanism of this embodiment includes but is not limited to the following structures: the second control mechanism includes a bending manipulation wire 104, one end of the bending manipulation wire is connected to a forceps base of the forceps head 101, the other end is located at a control end of the rigid biopsy forceps 100, and the bending of the elastic guide rod segment 102 is implemented by driving the bending manipulation wire 104, thereby implementing the steering of the forceps head 101. Optionally, the bending manipulation wire 104 located at the control end of the rigid biopsy forceps 100 is provided with a pulling ring 105, to facilitate pulling by the operator. Alternatively, the bending manipulation wire 104 is wound on a rotation winding mechanism, to implement the pulling and release of the bending manipulation wire 104 by rotating anticlockwise and clockwise.

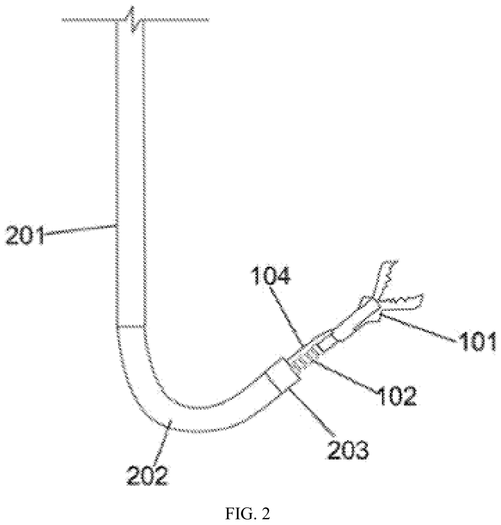

In this embodiment, the elastic guide rod segment 102 and the soft tube segment 202 both have a certain length. The elastic guide rod segment 102 has a first length, the soft tube segment 202 has a second length, and the specific lengths of both are set according to actual requirements. Optionally, the first length is greater than the second length, and the first length is 1.5 to 2 times the second length. It should be noted that in an actual operation process, a front end of the elastic guide rod segment 102 may extend out of an insertion head 203 or may not extend out of an insertion head 203, as long as the forceps head 101 can extend out of the insertion head 203. FIG. 2 only shows a situation where the front end of the elastic guide rod segment 102 extends out of the insertion head 203.

In this embodiment, the elastic guide rod segment 102 and the rigid guide rod segment 103 are of an integral structure, which is made by laser cutting or 3D printing. For example, the elastic guide rod segment 102 is a helical spring tube cut by a laser. During laser cutting, a front portion of one entire rigid guide rod is cut to form the elastic guide rod segment 101. One end of the elastic guide rod segment 101 is the rigid guide rod segment 103, and the other end further has a small segment of rigid guide rod segment, to facilitate connection with the forceps base 1011 of the forceps head. Cutting is performed along a peripheral surface of the rigid guide rod according to a thread line trajectory, and a structure of the made helical spring tube is similar to a spring structure. In one of the optional implementations, the helical spring tube has a pitch, and the pitch progressively decreases from a middle portion of the helical spring tube towards two ends. The helical spring tube has a large pitch in the middle portion, which is more prone to elastic deformation, so that the bending of the forceps head is more flexible. At the same time, pitches at the two ends are small, making it more difficult for the elastic deformation to occur. This can not only ensure a smooth movement of the forceps head in the tissue, but also allow for a gradual increase in a control force required when adjusting a direction of the forceps head. In this way, a steering direction of the forceps head can be controlled more precisely, to prevent the forceps head from being adjusted too flexibly in the direction and failing to precisely align with a required sampling position, which may lead to low acquisition efficiency.

In this embodiment, the forceps head 101 includes the forceps base 1011, a first forceps jaw 1012, and a second forceps jaw 1013, and the forceps base 1011 is fixedly connected to the elastic guide rod segment 102. The first forceps jaw 1012 and the second forceps jaw 1013 are disposed on the forceps base 1011 in an openable manner, and the first forceps jaw 1012 and the second forceps jaw 1013 form an accommodation space for accommodating the tissue after being opened.

In one of the implementations, the first forceps jaw 1012 is provided with first row teeth, and an end portion of the first row teeth is provided with a first end tooth 1012a. The second forceps jaw 1013 is provided with second row teeth, and an end portion of the second row teeth is provided with a second end tooth 1013a. In a fully open state of the first forceps jaw 1012 and the second forceps jaw 1013, the outermost end tooth of at least one forceps jaw can be inserted into the tissue at a vertical or near-vertical angle, which can improve the efficiency and effect of tissue acquisition.

For example, longitudinal cross sections of the first end tooth 1012a and the second end tooth 1013a are both triangles, a vertex angle of the first end tooth 1012a has a first angle bisector, and a vertex angle of the second end tooth 1013a has a second angle bisector. An included angle between the first forceps jaw 1012 and the second forceps jaw 1013 has a third angle bisector. In the fully open state of the first forceps jaw 1012 and the second forceps jaw 1013, included angles between the first angle bisector, the second angle bisector, and the third angle bisector are all α, where 0° ≤ α ≤ 5°. Through this disposed structure, when the first forceps jaw 1012 and the second forceps jaw 1013 are fully opened, the first end tooth 1012a at the outermost end of the first row teeth and the second end tooth 1013a at the outermost end of the second row teeth can be inserted into the tissue at the vertical or near-vertical angle, improving the efficiency and effect of tissue acquisition.

Further, the first row teeth on the first forceps jaw 1012 and the second row teeth on the second forceps jaw 1013 are uniformly arranged in a U shape or a ring shape. A middle portion of the first forceps jaw 1012 is provided with a first accommodation space that is concave, and the first row teeth are arranged around the first accommodation space. A middle portion of the second forceps jaw 1013 is provided with a second accommodation space that is concave, and the second row teeth are arranged around the second accommodation space. After the first forceps jaw 1012 and the second forceps jaw 1013 are engaged, all teeth of the first row teeth and the second row teeth or a part of teeth are seamlessly engaged, and the first accommodation space and the second accommodation space jointly constitute the accommodation space for accommodating the tissue. The tissue can be completely cut, preventing tissue tearing and secondary damage during removal.

Furthermore, at least one of the first forceps jaw 1012 and the second forceps jaw 1013 is provided with a liquid discharge hole, and the liquid discharge hole is communicated with the accommodation space. In other words, the first forceps jaw is provided with a first liquid discharge hole, and the first liquid discharge hole is communicated with the first accommodation space; and/or, the second forceps jaw is provided with a second liquid discharge hole, and the second liquid discharge hole is communicated with the second accommodation space. This disposed structure may discharge blood from clamped tissue and clamp more tissue, improving clamping efficiency.

In this embodiment, a front end of the soft tube segment 202 is provided with the insertion head 203, a front end of the insertion head 203 is provided with a camera 204 and a light source lamp 205, and the camera 204 and the light source lamp 205 are embedded into an end surface of a bottom portion of the insertion head 203.

In one of the optional implementations, the housing 207 includes a first housing 2071, a second housing 2072 is fixedly connected to a top portion of the first housing 2071, and the second housing 2072 is formed by a box body and a box cover snapping together. The rigid tube segment 201 is fixedly communicated with a bottom portion of the first housing 2071.

In this embodiment, a mounting frame 208 is disposed in the first housing 2071 of the housing 207, and the mounting frame 208 is internally provided with a guide tube 209 in a penetrating manner. The bottom portion of the insertion head 203 is provided with a through hole 2031 along a length direction thereof, and the through hole is communicated with the hollow channel of the endoscope tube. One end of the endoscope tube is communicated with the through hole, and the other end is communicated with the guide tube 209. The insertion portion of the rigid biopsy forceps 100 is coaxially inserted into the guide tube 209 and the hollow channel of the endoscope tube. The biopsy channel opening is disposed at a top portion of the second housing 2072, and the biopsy channel opening is provided with a mounting head 210 of a hollow structure. An opening at a top end of the mounting head 210 is detachably provided with a sealing plug 211, and the opening at the top end of the mounting head 210 overlaps with the central line of the biopsy channel opening. Optionally, the sealing plug 211 is threadedly connected to a top portion of the mounting head 210.

In this embodiment, the soft tube segment 202 of the thoracoscope 200 can synchronously bend with the elastic guide rod segment 102 of the rigid biopsy forceps 100, which belongs to passive bending. A bending angle of the soft tube segment 202 is mainly driven by the elastic guide rod segment 102. In an actual operation process, it is found that it is sometimes difficult to implement precise adjustment of the angle of the forceps head by only relying on the bending of the elastic guide rod segment 102. Therefore, in an optional implementation of this embodiment, the soft tube segment 202 can also actively bend, which can cooperate with the elastic guide rod segment 102 to bend, implementing more precise adjustment of the bending angle of the forceps head. Specifically, a fastening frame 212 is further disposed in an internal space of the housing 207, an adjustment disc 213 is fixedly connected to the fastening frame 212, a guide wire 214 is connected to the adjustment disc 213 in a wound manner, and two ends of the guide wire 214 are both connected to the insertion head 203. A bottom end of the fastening frame 212 is fixedly connected to the inside of the first housing 2071, and a top end of the fastening frame 212 extends into an internal space of the second housing 2072 above the housing 207. A handle 215 is disposed outside the housing 207, the handle 215 is connected to the adjustment disc 213, and the guide wire 214 is retracted and extended by rotating the adjustment disc 213 with the handle 215, thereby controlling the bending of the soft tube segment 202. In this way, the soft tube segment 202 can also actively bend, to assist in the bending of the elastic guide rod segment 102 of the rigid biopsy forceps. The bending of the soft tube segment 202 cooperates with the bending of the elastic guide rod segment 102, which allows for a wider and more precise range of angle adjustment for the forceps head.

In this embodiment, a soft tube 216 is further connected to a side wall of the mounting head 210, and the soft tube 216 is connected to an external pump body. A foreign object is sucked into the guide tube 209 through the through hole in a manner of negative pressure absorption and is discharged by the soft tube 216, thereby discharging the foreign object.

In this embodiment, the operating main body 206 is further provided with a data interface 217, and the data interface 217 is disposed on one side of the second housing 2072. The data interface 217 is connected to a computer through a data cable, and a data image of an operation process is transmitted to the computer through the data cable.

In one of the optional implementations, the third control mechanism includes a first key 218 and a second key, and the first key 218 and the second key 219 are disposed on a front surface of the second housing 2072. The first key 218 is used for shooting an internal image of a thoracic cavity in real time, facilitating the search for the position of the foreign object. The second key 219 is used for storing shooting data, facilitating timely invoking and viewing.

Compared with the prior art, the flexible thoracoscope kit with a straight working channel provided in this embodiment can at least implement one of the following beneficial effects:
1. The insertion portion of the forceps head of the rigid biopsy forceps includes the elastic guide rod segment and the rigid guide rod segment. The endoscope tube of the thoracoscope includes the rigid tube segment and the soft tube segment. The soft tube segment bends synchronously by controlling the bending of the elastic guide rod segment. This can conveniently and rapidly adjust the angle of the forceps head, to acquire a large tissue standard block sample, thereby improving the diagnosis and treatment efficacy under the flexible thoracoscope.
2. The biopsy channel of the thoracoscope is designed in a penetrating manner, and the biopsy channel opening thereof is in the center of the top portion of the operating main body. On the one hand, the rigid biopsy forceps and therapeutic tools can pass through the front flexible thoracoscope, improving the capability of grasping diseased tissue during biopsy and treatment. On the other hand, better coaxiality between the flexible rigid biopsy forceps and the thoracoscope body is ensured, reducing the impact of the operator rotating a wrist to adjust the thoracoscope body of the thoracoscope on the operation of the flexible rigid biopsy forceps. In addition, this has the advantage of a small single-port trauma, which has better effects than that of biopsy and treatment using traditional rigid tools.
3. The thoracoscope with a straight working channel can be compatible with a flexible biopsy/treatment tool and a rigid biopsy/treatment tool, both of which can pass through. The operating habits are similar to those of a traditional flexible thoracoscope, which are easy to learn and master. The addition of the rigid biopsy/treatment tool for passing through makes the biopsy and treatment efficacy thereof similar to that of a rigid thoracoscope, compensating for the insufficient efficacy of the flexible thoracoscope and the flexible biopsy/treatment tool.
4. When the first forceps jaw and the second forceps jaw of the forceps head are in the fully open state, the outermost end tooth of at least one forceps jaw can be inserted into the tissue at a vertical or near-vertical angle, which can improve the efficiency and effect of tissue acquisition.
5. The soft tube segment of the thoracoscope can also actively bend, which can cooperate with the elastic guide rod segment to bend, implementing more precise adjustment of the bending angle of the forceps head.

In the above specific implementations, the purposes, technical solutions, and beneficial effects of this disclosure are further described in detail. It should be understood that the above descriptions are merely specific implementations of this disclosure, but are not intended to limit the protection scope of this disclosure. Any modification, equivalent replacement, improvement, or the like made without departing from the spirit and principle of this disclosure shall fall within the protection scope of this disclosure.

## Claims

1. A flexible thoracoscope kit with a straight working channel, comprising:
rigid biopsy forceps, wherein the rigid biopsy forceps are provided with a forceps head, an elastic guide rod segment, and a rigid guide rod segment, the forceps head is connected to a first end of the rigid guide rod segment through the elastic guide rod segment, a second end of the rigid guide rod segment is connected to a first control mechanism and a second control mechanism, the first control mechanism is configured to control opening and closing of the forceps head, and the second control mechanism is configured to control bending of the elastic guide rod segment, to implement steering of the forceps head; and
a thoracoscope, wherein the thoracoscope is provided with an endoscope tube, and the endoscope tube is provided with a hollow channel extending along an axial direction; and the endoscope tube comprises a rigid tube segment and a soft tube segment, and a rear end of the soft tube segment is connected to a front end of the rigid tube segment; and
the forceps head, the elastic guide rod segment, and the rigid guide rod segment are movably mounted in the hollow channel, the elastic guide rod segment at least partially overlaps with the soft tube segment, and the soft tube segment can synchronously bend with the elastic guide rod segment.

2. The flexible thoracoscope kit with a straight working channel according to claim 1, wherein the second control mechanism comprises a bending manipulation wire, one end of the bending manipulation wire is connected to a forceps base of the forceps head, the other end is located at a control end of the rigid biopsy forceps, and the bending of the elastic guide rod segment is implemented by driving the bending manipulation wire, thereby implementing the steering of the forceps head.

3. The flexible thoracoscope kit with a straight working channel according to claim 2, wherein the elastic guide rod segment and the rigid guide rod segment are of an integral structure, and the elastic guide rod segment is a helical spring tube cut by a laser.

4. The flexible thoracoscope kit with a straight working channel according to claim 1, wherein the forceps head comprises a forceps base, a first forceps jaw, and a second forceps jaw, and the forceps base is fixedly connected to the elastic guide rod segment; and the first forceps jaw and the second forceps jaw are disposed on the forceps base in an openable manner, and the first forceps jaw and the second forceps jaw form an accommodation space for accommodating tissue after being opened.

5. The flexible thoracoscope kit with a straight working channel according to claim 4, wherein the first forceps jaw is provided with first row teeth, and an end portion of the first row teeth is provided with a first end tooth; and the second forceps jaw is provided with second row teeth, and an end portion of the second row teeth is provided with a second end tooth;
longitudinal cross sections of the first end tooth and the second end tooth are both triangles, a vertex angle of the first end tooth has a first angle bisector, and a vertex angle of the second end tooth has a second angle bisector;
an included angle between the first forceps jaw and the second forceps jaw has a third angle bisector; and
in a fully open state of the first forceps jaw and the second forceps jaw, included angles between the first angle bisector, the second angle bisector, and the third angle bisector are all α, wherein 0° ≤ α ≤ 5°.

6. The flexible thoracoscope kit with a straight working channel according to claim 1, wherein a front end of the soft tube segment is provided with an insertion head, and a front end of the insertion head is provided with a camera and a light source lamp.

7. The flexible thoracoscope kit with a straight working channel according to claim 6, wherein the thoracoscope is further provided with an operating main body, the operating main body is provided with a housing, a bottom portion of the housing is provided with a mounting opening, and the endoscope tube is connected to the mounting opening in a sealing manner; and a center of a top portion of the housing is provided with a biopsy channel opening, and a central line of the biopsy channel opening overlaps with an axis of the rigid tube segment.

8. The flexible thoracoscope kit with a straight working channel according to claim 7, wherein the operating main body is provided with a third control mechanism, and the third control mechanism is configured to control a normal operation of the thoracoscope.

9. The flexible thoracoscope kit with a straight working channel according to claim 7, wherein a mounting frame is disposed in a lower space of the housing, and the mounting frame is internally provided with a guide tube in a penetrating manner; a bottom portion of the insertion head is provided with a through hole along a length direction thereof, and the through hole is communicated with the hollow channel of the endoscope tube; and one end of the endoscope tube is communicated with the through hole, and the other end is communicated with the guide tube.

10. The flexible thoracoscope kit with a straight working channel according to claim 7, wherein the biopsy channel opening at the top portion of the housing is provided with a mounting head of a hollow structure, and an opening at a top end of the mounting head is detachably provided with a sealing plug.
